# EUROPEAN PATENT APPLICATION

(11) **EP 4 000 658 A1**
(43) Date of publication of application: **25.05.2022**
(21) Application number: 21181183.1
(22) Date of filing: 23.06.2021
(51) Int. Cl.: A61L 27/20, A61L 27/36, B33Y 10/00

(54) **BONE GRAFT COMPOSITION**

(30) Priority: 20.11.2020 KR 20200156748
(71) Applicant: MedPark Co., Ltd., Busan 46504 (KR)
(72) Inventor: PARK, Jung Bok, 46509 Busan (KR)
(74) Representative: BCKIP

(57) **Abstract**

The present disclosure relates to a bone graft composition containing a bone graft material and an additive and having a predetermined viscosity, wherein the additive includes only a methylcellulose-based additive, and a viscosity of the bone graft composition is 100 to 500,000 centipoises (cps).

## Description

### CROSS-REFERENCE TO RELATED APPLICATION

This application claims priority to Korean Patent Application No. 10-2020-0156748, filed on November 20, 2020.

### BACKGROUND

### 1. Technical Field

The present disclosure relates to a bone graft composition.

### 2. Related Art

Various materials and various methods may be used for reconstruction of defective bone. For example, bone graft materials such as bone powders, bone chips, and bone blocks may be used, or methods such as autografting, allografting, and xerografting may be used for reconstruction of defective bone.

Bone graft materials that are used for reconstruction of defective bone may be used in general surgery, orthopedic surgery, neurosurgery, plastic surgery, thoracic surgery, otolaryngology, oral and maxillofacial surgery, veterinary medicine (veterinary hospital), dermatology, and dentistry. For example, these materials may be used for bone defects during disc surgery to induce bone regeneration, or may also be used for implant surgery and reconstruction of oral and maxillofacial bone defects.

In addition, the bone graft material may be used for reconstruction of defective bone in humans or animals. In the present disclosure, description will be made mainly of the case in which the bone graft material is used in dentistry, but the description is not limited thereto.

Meanwhile, Korean Patent No. 10-1443814 discloses a bone graft composition containing a hydrogel and calcium phosphate compound particles, and a preparation method therefor. When this composition is used, there may be limitations in the effect of bone grafting in terms of biocompatibility, viscosity, shape retainability, and the like.

### [Prior Art Documents]

### [Patent Documents]

(Patent Document 0001) Korean Patent No. 10-1443814

### SUMMARY

The present disclosure is directed to a bone graft composition, and an object of the present disclosure is to provide a bone graft composition containing a bone graft material and an additive and having adjusted viscosity, wherein the additive includes only a methylcellulose-based additive and the viscosity of the bone graft composition is 100 to 500,000 centipoises (cps).

Another object of the present disclosure is to provide a bone graft composition having excellent shape retainability while having adjusted viscosity.

Objects of the present disclosure are not limited to the above-mentioned objects, and other objects not mentioned herein will be clearly understood by those skilled in the art from the following description.

To achieve the above objects, the present disclosure provides a bone graft composition containing a bone graft material and an additive and having adjusted viscosity, wherein the additive includes only a methylcellulose-based additive and the viscosity of the bone graft composition is 100 to 500,000 centipoise (cps).

In one embodiment, the methylcellulose-based additive may be one or more of methylcellulose, carboxymethyl cellulose, and hydroxypropyl methylcellulose.

In one embodiment, the methylcellulose-based additive may be hydroxypropyl methylcellulose.

In one embodiment, the methylcellulose-based additive may include only one of methylcellulose, carboxymethyl cellulose, and hydroxypropyl methylcellulose, and the additive may be contained in an amount of 0.03 to 3 parts by weight based on 1 part by weight of the bone graft material so that the viscosity of the bone graft composition is 100 to 500,000 cps.

In one embodiment, the methylcellulose-based additive may be hydroxypropyl methylcellulose.

The present disclosure also provides a bone graft composition containing a bone graft material and an additive and having adjusted viscosity, wherein the additive includes one of methylcellulose, carboxymethyl cellulose, and hydroxypropyl methylcellulose, and the viscosity of the bone graft composition is adjusted depending on the amount of the additive and is 100 to 500,000 centipoise (cps).

In one embodiment, the additive may be hydroxypropyl methylcellulose.

In one embodiment, the additive may be contained in an amount of 0.03 to 3 parts by weight based on 1 part by weight of the bone graft material so that the viscosity of the bone graft composition is 100 to 500,000 cps.

The present disclosure also provides a bone graft composition containing a bone graft material and an additive and having adjusted viscosity, the composition having shape retainability of 50 or more, wherein the shape retainability is defined as a value obtained by dividing a maximum breaking force (Nmax) by a short-axis change rate, in which the maximum breaking force is a force at which a change in the shape of a sphere-shaped material is initiated by applying a force (N) to one side of the sphere-shaped material, and the short-axis change rate is the ratio (D-S/D) of the reduction in short-axis length (S) of the sphere-shaped material, measured after the change in the shape occurred, to the diameter (D) of the sphere-shaped material, and wherein the viscosity of the composition is adjusted depending on the amount of the additive and is 100 to 500,000 centipoise (cps).

In one embodiment, and the additive is hydroxypropyl methylcellulose.

### BRIEF DESCRIPTION OF THE DRAWINGS

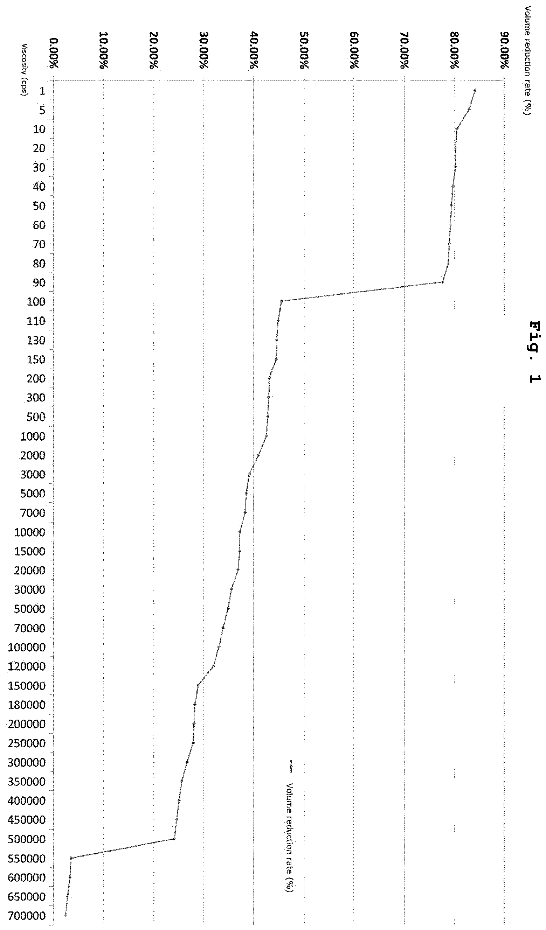

FIG. 1 shows the volume reduction rate of a mixture of a bone graft material and HPMC depending on the viscosity thereof, calculated based on the result data (Table 1) obtained in Experimental Example 1 of the present disclosure.

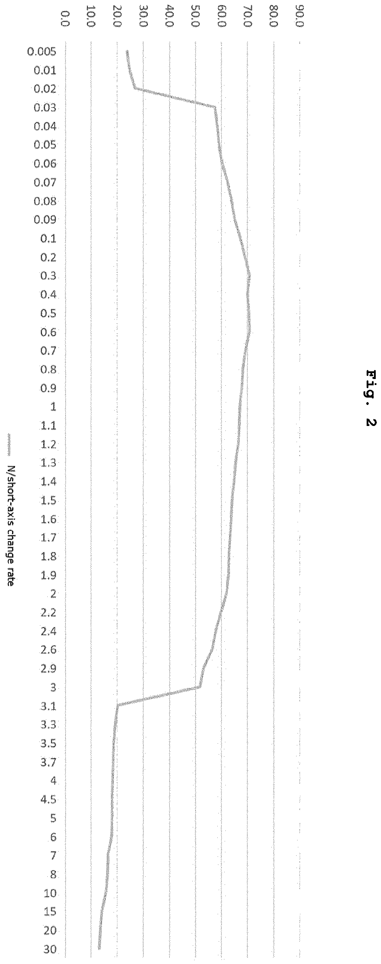

FIG. 2 shows "maximum breaking force (N)/short-axis change rate" of a mixture of a bone graft material and HPMC depending on the content (parts by weight) of HPMC at a viscosity ranging from 100 cps to 500,000 cps, calculated based on the result data (Table 2) obtained in Experimental Example 2 of the present disclosure.

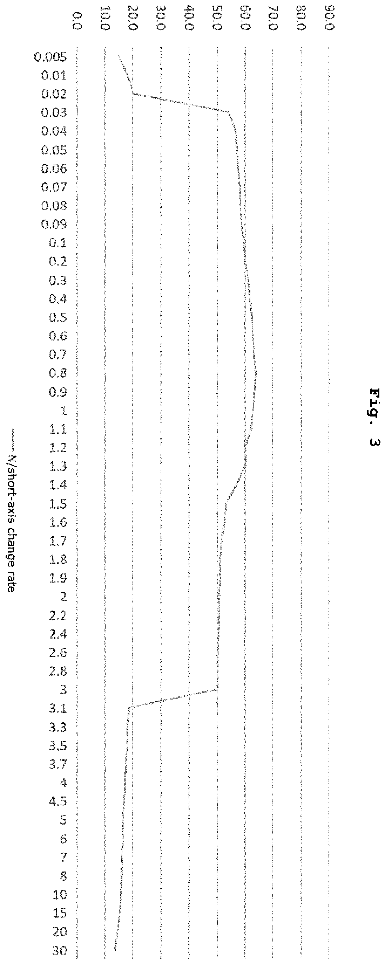

FIG. 3 shows "maximum breaking force (N)/short-axis change rate" of a mixture of a bone graft material and CMC depending on the content (parts by weight) of CMC at a viscosity ranging from 100 cps to 500,000 cps, calculated based on the result data (Table 3) obtained in Experimental Example 3 of the present disclosure.

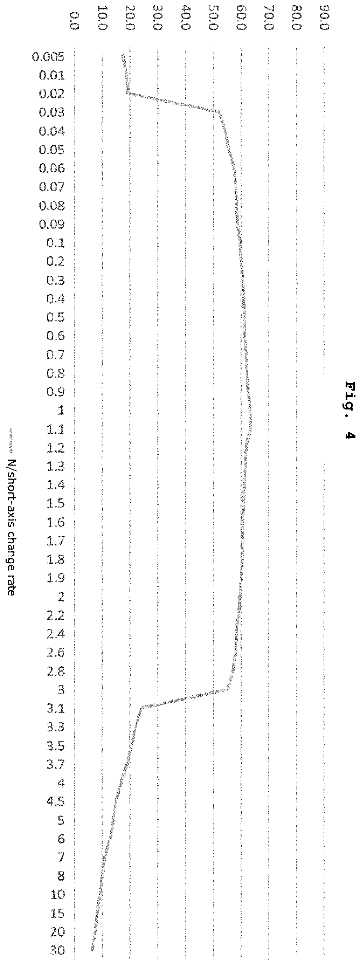

FIG. 4 shows "maximum breaking force (N)/short-axis change rate" of a mixture of a bone graft material and MC depending on the content (parts by weight) of MC at a viscosity ranging from 100 cps to 500,000 cps, calculated based on the result data (Table 4) obtained in Experimental Example 4 of the present disclosure.

### DETAILED DESCRIPTION

Embodiments of the present disclosure relate to a bone graft composition which may have adjusted viscosity and excellent effects in terms of activation of bone formation, biocompatibility, and ease of use by containing a bone graft material and an additive.

However, description of a portion of a particular embodiment, which overlaps with that of other embodiments, will be omitted for a clearer and more concise explanation. Even though description of that portion is omitted, the portion is not excluded from the present disclosure and the scope of rights thereof should be admitted in the same manner as that of other embodiments.

In the following description, the detailed description of publicly-known technology related to the present disclosure will be omitted when it may unnecessarily obscure the subject matter of the present disclosure. In addition, the terms used in the following description are terms defined in consideration of their functions in the present disclosure and may be changed according to the intention of a user or an operator, or according to practice. Therefore, the definitions of these terms should be determined based on the contents throughout the specification.

The technical spirit of the present disclosure is determined by the claims, and the following embodiments are merely means for efficiently explaining the technical spirit of the present disclosure to those skilled in the art to which the present disclosure pertains.

Hereinafter, specific embodiments of the present disclosure will be described. However, these embodiments are only examples, and the present disclosure is not limited thereto.

A bone graft composition according to one embodiment of the present disclosure may contain a methylcellulose-based additive. The bone graft composition may be implanted into a bone defect, and may be used to restore the bone defect by filling the bone defect.

As used herein, the term "filling" is meant to include not only a case where the bone graft composition is applied to a bone defect in a state in which it does not have rigidity, but also a case where the bone graft composition is applied to a bone defect in a state in which it has rigidity. The expression "applying the bone graft composition to a bone defect after imparting rigidity thereto" may mean applying the bone graft composition to the bone defect after preparing the composition in a rigid form corresponding to the bone defect by means of a shape forming device (e.g., a 3D printer, etc.). The bone graft material may be natural bone. For example, it may be autogenous bone, allogeneic bone, or xenogenic bone. When the natural bone is used, it may exhibit an excellent bone formation effect, because it has excellent biocompatibility and also has good wettability and hygroscopicity due to a large number of pores contained therein. In addition, the natural bone may also be used for reconstruction of defective bone in general surgery, orthopedic surgery, neurosurgery, plastic surgery, thoracic surgery, otolaryngology, oral and maxillofacial surgery, veterinary medicine (veterinary hospital), dermatology, and dentistry.

In addition, the bone graft material may be used for reconstruction of defective bone in humans or animals. In the present disclosure, description will be made mainly of the case in which the bone graft material is used in dentistry, but the description is not limited thereto.

As the bone graft composition contains the additive, the bone graft composition may have adhesion to a bone defect. At the same time, shape retainability may be imparted to the bone graft composition by the additive. When the bone graft composition has excellent shape retainability, even if the bone graft composition is applied to the maxilla, it may be applied to fit the bone defect without flowing down, and thus even if there is an impact due to mastication motion or the like, the bone graft composition may be prevented from being detached from the bone defect.

In the bone graft composition according to one embodiment of the present disclosure, a mixture of the bone graft material and the additive may be formed to have a viscosity of 100 to 500,000 cps. If the viscosity of the mixture of the bone graft material and the additive is lower than 100 cps, the strength of a hydrogel surrounding the bone graft material may be lowered, and the hydrogel cannot cover the bone graft material because the water content thereof is low. On the other hand, if the viscosity of the mixture of the bone graft material and the additive is higher than 500,000 cps, a problem may arise in that the shape retainability of the mixture is lowered, resulting in a change in the shape of the mixture. For this reason, the viscosity of the mixture of the bone graft material and the additive is between 100 and less than 500,000 cps. In this case, the hydrogel surrounding the bone graft material may have high strength and high water content and may harden, and thus the shape thereof may be set. Therefore, in order for the mixture of the bone graft material and the additive to have excellent shape retainability and also have optimal effects in term of activation of bone formation, biocompatibility, and ease of use, the bone graft material is preferably mixed only with a methylcellulose-based additive so that the mixture may have a viscosity between 100 cps and less than 500,000 cps.

The methylcellulose-based additive may include one or more selected from the group consisting of methylcellulose (MC), carboxymethyl cellulose (CMC), and hydroxypropyl methylcellulose (HPMC). In one embodiment of the present disclosure, the additive may be one or more of HPMC, CMC or MC, but is not limited thereto, and any additive may be used without limitation as long as it is a methylcellulose-based additive as mentioned above. The additive may be contained in an amount of 0.03 to 3 parts by weight based on 1 part by weight of the bone graft material so that the viscosity of the bone graft composition is 100 to 500,000 cps. When the additive is contained in an amount of 0.03 to 3 parts by weight based on 1 part by weight of the bone graft material and the viscosity of the bone graft composition is 100 to 500,000 cps, the shape retainability of the bone graft composition may be 50 or more.

In the additive and bone graft composition according to one embodiment of the present disclosure, the additive may be contained in an amount of 0.03 to 3 parts by weight based on 1 part by weight of the bone graft material in order to ensure the shape retainability of the bone graft composition, and in this case, the shape retainability is further enhanced. As can be seen from the Experimental Examples to be described below, when the content of the additive is 0.03 to 3 parts by weight, the short-axis change rate increases, and when the content of the additive is less than 0.03 and more than 3 parts by weight, the short-axis change rate decreases. As shown in FIG. 2 to 4, it can be seen that this increase and decrease are abrupt. Thereby, it has been found that the content of the additive should be 0.03 parts by weight to 3 parts by weight based on 1 part by weight of the bone graft material in order to provide the bone graft composition intended by the present disclosure. If the content of the additive is less than 0.03 parts by weight based on 1 part by weight of the bone graft material, the effect of adding the additive may be insignificant due to an excessively low specific gravity of the additive, and hence the bone graft material may be easily pressed and broken even by low pressure (force), so that the shape of the bone graft material cannot be suitably retained. If the content of the additive is more than 3 parts by weight based on 1 part by weight of the porous bone graft material, the bone graft material can be easily pressed even by a small force due to an excessively high specific gravity of the additive and the short-axis change rate can also increase, so that the shape of the bone graft material cannot be suitably retained.

The shape retainability may be defined as a value obtained by dividing a maximum breaking force (Nmax) by a short-axis change rate, in which the maximum breaking force (Nmax) is a force at which a change in shape of a sphere-shaped material is initiated by applying a force (N) to one side of the sphere-shaped material, and the short-axis change rate is a ratio (D-S)/(D) of a reduction in short-axis length (S) of the sphere-shaped material, measured after the change in the shape occurred, to a diameter (D) of the sphere-shaped material.

The bone graft composition prepared to contain the additive and the bone graft material as described above should have an ensured hydration ability for application of the composition to the human body, for example, application of the composition to teeth. In other words, the bone graft composition should have critical osmotic properties. Allowing the bone graft composition to form a certain osmotic ratio with another solution (e.g., saline) is an important factor in the ability of the bone graft material to be maintained at a constant concentration to thereby retain its shape and function. This property may be determined depending on the content of the additive contained in the bone graft composition, as well as the condition of a solvent for forming a solution of the composition.

For example, in the case in which the bone graft composition is applied to teeth, a dental operator applies the bone graft composition, which is provided as powder or the like, to a missing tooth after hydrating the composition. If the additive contained in the bone graft composition flows out in large amounts (that is, the osmotic pressure of the hydrated composition increases) during this hydration process, the additive cannot remain inside the bone graft material, and thus the bone graft material cannot retain its shape and also cannot agglomerate, making it impossible to perform the medical procedure. For this reason, the bone graft composition should have osmotic properties within a predetermined range so that it can retain its shape without being influenced by external conditions. Furthermore, if an external environment such as water or saliva is formed after the bone graft composition is applied to the missing tooth, a phenomenon may occur in which the bone graft composition flows out around or detaches, and thus a problem may arise in that an external substance flows into the missing tooth. For this reason, the bone graft composition should have osmotic properties within a predetermined range, and furthermore, have not only internal physical properties, but also properties that resist external conditions.

### Experimental Example 1

### 1. Experiment for Examining the Volume Reduction Rate of Mixture of Bone Graft Material and Hydroxypropyl Methylcellulose (HPMC) depending on the Viscosity Thereof

A bone graft material and HPMC were tested at various viscosities (0 to 700,000 cps as shown in Table 1 below. A mixture of a bone graft material and HPMC was dissolved in a solvent to prepare gel-type bone graft materials, and the initial volume (A) and volume after 48 hours (B) of each gel-type bone graft material were measured. The volume reduction rate (%) was calculated using the following equation: volume reduction rate (%) = volume after 48 hours (B) - initial volume (A) / 100.

Specifically, each of the hydrated samples was placed closely in a 15-ml conical tube, and then the initial volume was measured. Then, a portion of the conical tube, not occupied by the sample, was cut out. Next, the opening of the conical tube remaining after cutting was covered with a mesh so that only the dissolved sample could pass through the opening. Thereafter, the conical tube was placed in an ultrasonic cleaner maintained at the human body temperature (e.g., 37°C), and purified water was circulated therethrough at a constant rate. After 48 hours, the volume of the sample was measured. Before the volume of the sample was measured, purified water remaining in the sample could be removed.

The volume reduction rate of the mixture of the bone graft material and the additive in the environment similar to that of the human body was measured depending on the viscosity thereof. It can be understood that the larger the volume reduction rate, the greater the solubility of the sample, and the smaller the volume reduction rate, the smaller the solubility.

The amount of the solvent (water) is the optimal amount of hydration in which the mixture can dissolve well. The amount of the solvent may be 0.5 to 2 times the total weight of the mixture. In this Example, the amount of the solvent was 1.2 times the total weight of the mixture.

**[Table 1]**

| Viscosity (cps) of bone graft material + additive | Initial volume (cc) | Volume (cc) after 48 hours | Volume reduction rate |
|---|---|---|---|
| **1** | 0.330 | 0.034 | 89.70% |
| **5** | 0.340 | 0.037 | 89.12% |
| **10** | 0.350 | 0.040 | 88.57% |
| **20** | 0.360 | 0.043 | 88.06% |
| **30** | 0.370 | 0.045 | 87.84% |
| **40** | 0.380 | 0.050 | 86.84% |
| **50** | 0.390 | 0.053 | 86.41% |
| **60** | 0.400 | 0.057 | 85.75% |
| **70** | 0.410 | 0.060 | 85.37% |
| **80** | 0.420 | 0.062 | 85.24% |
| **90** | 0.520 | 0.078 | 85.00% |
| **100** | 2.220 | 0.801 | 63.92% |
| **110** | 2.320 | 0.840 | 63.79% |
| **130** | 2.420 | 0.882 | 63.55% |
| **150** | 2.520 | 0.920 | 63.49% |
| **200** | 2.620 | 0.960 | 63.36% |
| **300** | 2.720 | 1.002 | 63.16% |
| **500** | 2.820 | 1.050 | 62.77% |
| **1000** | 2.920 | 1.090 | 62.67% |
| **2000** | 3.020 | 1.130 | 62.58% |
| **3000** | 3.120 | 1.170 | 62.50% |
| **5000** | 3.220 | 1.215 | 62.27% |
| **7000** | 3.320 | 1.269 | 61.78% |
| **10000** | 3.420 | 1.310 | 61.70% |
| **15000** | 3.520 | 2.161 | 61.40% |
| **20000** | 3.620 | 2.173 | 60.02% |
| **30000** | 3.720 | 2.350 | 58.98% |
| **50000** | 3.820 | 2.410 | 57.97% |
| **70000** | 3.920 | 2.620 | 56.86% |
| **100000** | 4.020 | 2.788 | 55.02% |
| **120000** | 4.120 | 2.912 | 29.32% |
| **150000** | 4.220 | 3.002 | 28.86% |
| **180000** | 4.320 | 3.103 | 28.17% |
| **200000** | 4.420 | 3.180 | 28.05% |
| **250000** | 4.520 | 3.260 | 27.88% |
| **300000** | 5.020 | 3.680 | 26.69% |
| **350000** | 5.120 | 3.810 | 25.59% |
| **400000** | 5.220 | 3.912 | 25.06% |
| **450000** | 5.320 | 4.012 | 24.59% |
| **500000** | 5.420 | 4.112 | 24.13% |
| **550000** | 5.520 | 5.327 | 3.50% |
| **600000** | 5.620 | 5.436 | 3.27% |
| **650000** | 5.720 | 5.560 | 2.80% |
| **700000** | 5.820 | 5.680 | 2.41% |

As shown in Table 1 above, it can be confirmed that, as the viscosity of the mixture of the bone graft material and the additive increased from 0 cps to 700,000 cps, the volume reduction rate (%) of the mixture decreased. In addition, it can be confirmed that the volume reduction rate rapidly decreased in a specific viscosity range. In particular, it can be confirmed that the volume reduction rate of the mixture of the bone graft material and the additive rapidly decreased by 20% or more in a viscosity range of 90 cps to 100 cps and in a viscosity range of 500,000 cps to 550,000 cps.

FIG. 1 shows changes in the volume reduction rate of the mixture of the bone graft material and the additive depending on the viscosity thereof, calculated based on the result data (Table 1) obtained in Experimental Example 1. In the graph of FIG. 1, a value on the X-axis, which is closer to 0, means that the mixture of the bone graft material and the additive is not viscous, and a value on the Y-axis, which is closer to 0, means that the change in the volume after 48 hours of the mixture is not significant. Here, the volume after 48 hours may be defined as the time until the bone graft material is stabilized in the patient's bone defect after a doctor applies the bone graft material to the patient.

### Experimental Example 2

### 2. Experiment for Examining Shape Retainability Using "Maximum Breaking Force (N)/Short-Axis Change Rate" Depending on the Content (Parts by Weight) of HPMC in the Viscosity Range of 100 cps to 500,000 cps

In order to form a bone graft composition having excellent shape retainability, optimal strength and non-resilience are required. Table 2 below shows the maximum breaking force relative to the short-axis change rate depending on the content of HPMC. FIG. 2 shows "maximum breaking force (N) of bone graft composition sphere/short-axis change rate of bone graft composition sphere" as a function of the content (parts by weight) of HPMC in the mixture of the bone graft material and HPMC in the viscosity range of 100 cps to 500,000 cps, calculated based on the values shown in Table 2.

In FIG. 2, the X-axis shows different amounts (0.005 to 30 parts by weight) of HPMC added per part by weight of the bone graft material, and the Y-axis shows the values of "maximum breaking force (N)/short-axis change rate" as a function of the amounts of HPMC added. The shape retainability of each of the resulting mixtures as a function of the content (parts by weight) of HPMC in the viscosity range of 100 cps to 500,000 cps was examined.

**[Table 2]**

| Content (parts by weight of HPMC) | N/short-axis change rate |
|---|---|
| 0.005 | 23.8 |
| 0.01 | 24.7 |
| 0.02 | 26.8 |
| 0.03 | 57.4 |
| 0.04 | 58.5 |
| 0.05 | 59.1 |
| 0.06 | 60.2 |
| 0.07 | 62.3 |
| 0.08 | 63.8 |
| 0.09 | 65.0 |
| 0.1 | 67.2 |
| 0.2 | 69.1 |
| 0.3 | 70.8 |
| 0.4 | 70.1 |
| 0.5 | 70.6 |
| 0.6 | 70.9 |
| 0.7 | 69.2 |
| 0.8 | 68.3 |
| 0.9 | 67.8 |
| 1 | 67.1 |
| 1.1 | 66.8 |
| 1.2 | 66.5 |
| 1.3 | 65.4 |
| 1.4 | 64.8 |
| 1.5 | 64.1 |
| 1.6 | 63.7 |
| 1.7 | 63.2 |
| 1.8 | 62.9 |
| 1.9 | 62.7 |
| 2 | 61.9 |
| 2.2 | 59.8 |
| 2.4 | 57.8 |
| 2.6 | 56.5 |
| 2.9 | 53.2 |
| 3 | 51.8 |
| 3.1 | 20.3 |
| 3.3 | 19.3 |
| 3.5 | 18.7 |
| 3.7 | 18.5 |
| 4 | 18.2 |
| 4.5 | 18.1 |
| 5 | 18.0 |
| 6 | 17.8 |
| 7 | 16.5 |
| 8 | 16.2 |
| 10 | 15.8 |
| 15 | 14.2 |
| 20 | 13.5 |
| 30 | 13.2 |

As shown in Table 2 above, when the content of HPMC was less than 0.03 parts by weight and more than 3.0 parts by weight, the short-axis change rate was high and the maximum breaking force (N) was low. Thus, in this case, the "maximum breaking force (N)/short-axis change rate" value was low, and hence the shape retainability was not excellent. This means that the shape is easily changed even with a slight force, and this change is very unfavorable during a medical procedure for application of the bone graft composition.

When HPMC was contained in an amount of 0.3 to 3 parts by weight based on 1 part by weight of the bone graft material, it was possible to obtain a bone graft composition having excellent shape retainability due to a "maximum breaking force (N)/short-axis change rate" of 50 or more.

### Experimental Example 3

### 3. Experiment for Examining Shape Retainability Using "Maximum Breaking Force (N)/Short-Axis Change Rate" Depending on the Content (Parts by Weight) of Carboxymethyl Cellulose (CMC) in the Viscosity Range of 100 cps to 500,000 cps

Like Experimental Example 2, Table 3 below shows the maximum breaking force relative to the short-axis change rate depending on the content of CMC. FIG. 3 shows "maximum breaking force (N) of bone graft composition sphere/short-axis change rate of bone graft composition sphere" as a function of the content (parts by weight) of CMC in the mixture of the bone graft material and CMC in the viscosity range of 100 cps to 500,000 cps, calculated based on the values shown in Table 3.

In FIG. 3, the X-axis shows different amounts (0.005 to 30 parts by weight) of CMC added per part by weight of the bone graft material, and the Y-axis shows the values of "maximum breaking force (N)/short-axis change rate" as a function of the amounts of CMC added. The shape retainability of each of the resulting mixtures as a function of the content (parts by weight) of CMC in the viscosity range of 100 cps to 500,000 cps was examined.

**[Table 3]**

| Content (parts by weight of CMC) | N/short-axis change rate |
|---|---|
| 0.005 | 15.0 |
| 0.01 | 18.2 |
| 0.02 | 20.3 |
| 0.03 | 54.2 |
| 0.04 | 56.7 |
| 0.05 | 57.2 |
| 0.06 | 57.6 |
| 0.07 | 58.2 |
| 0.08 | 58.5 |
| 0.09 | 58.9 |
| 0.1 | 59.6 |
| 0.2 | 60.2 |
| 0.3 | 61.1 |
| 0.4 | 61.8 |
| 0.5 | 62.5 |
| 0.6 | 62.9 |
| 0.7 | 63.2 |
| 0.8 | 63.9 |
| 0.9 | 63.5 |
| 1 | 62.9 |
| 1.1 | 62.3 |
| 1.2 | 60.2 |
| 1.3 | 60.1 |
| 1.4 | 57.1 |
| 1.5 | 53.4 |
| 1.6 | 52.7 |
| 1.7 | 51.7 |
| 1.8 | 51.3 |
| 1.9 | 51.1 |
| 2 | 50.9 |
| 2.2 | 50.7 |
| 2.4 | 50.6 |
| 2.6 | 50.3 |
| 2.8 | 50.3 |
| 3 | 50.1 |
| 3.1 | 18.7 |
| 3.3 | 18.2 |
| 3.5 | 18.1 |
| 3.7 | 17.5 |
| 4 | 17.2 |
| 4.5 | 16.8 |
| 5 | 16.5 |
| 6 | 16.4 |
| 7 | 16.2 |
| 8 | 16.1 |
| 10 | 15.8 |
| 15 | 15.3 |
| 20 | 14.5 |
| 30 | 13.8 |

As shown in Table 3 above, when the content of CMC was less than 0.03 parts by weight and more than 3.0 parts by weight, the short-axis change rate was high and the maximum breaking force (N) was low. Thus, in this case, the "maximum breaking force (N)/short-axis change rate" value was low, and hence the shape retainability was not excellent. This means that the shape is easily changed even with a slight force, and this change is very unfavorable during a medical procedure for application of the bone graft composition.

When CMC was contained in an amount of 0.03 to 3 parts by weight based on 1 part by weight of the bone graft material, it was possible to obtain a bone graft composition having excellent shape retainability due to a "maximum breaking force (N)/short-axis change rate" of 50 or more.

### Experimental Example 4

### 4. Experiment for Examining Shape Retainability Using "Maximum Breaking Force (N)/Short-Axis Change Rate" Depending on the Content (Parts by Weight) of Methylcellulose (MC) in the Viscosity Range of 100 cps to 500,000 cps

Like Experimental Example 2, Table 4 below shows the maximum breaking force relative to the short-axis change rate depending on the content of MC. FIG. 4 shows "maximum breaking force (N) of bone graft composition sphere/short-axis change rate of bone graft composition sphere" as a function of the content (parts by weight) of MC in the mixture of the bone graft material and MC in the viscosity range of 100 cps to 500,000 cps, calculated based on the values shown in Table 4.

In FIG. 4, the X-axis shows different amounts (0.005 to 30 parts by weight) of MC added per part by weight of the bone graft material, and the Y-axis shows the values of "maximum breaking force (N)/short-axis change rate" as a function of the amounts of MC added. The shape retainability of each of the resulting mixtures as a function of the content (parts by weight) of MC in the viscosity range of 100 cps to 500,000 cps was examined.

**[Table 4]**

| Content (parts by weight of MC) | N/short-axis change rate |
|---|---|
| 0.005 | 17.5 |
| 0.01 | 18.7 |
| 0.02 | 19.2 |
| 0.03 | 52.1 |
| 0.04 | 54.3 |
| 0.05 | 55.7 |
| 0.06 | 57.6 |
| 0.07 | 58.2 |
| 0.08 | 58.5 |
| 0.09 | 58.9 |
| 0.1 | 59.6 |
| 0.2 | 60.1 |
| 0.3 | 60.5 |
| 0.4 | 60.9 |
| 0.5 | 61.2 |
| 0.6 | 61.4 |
| 0.7 | 61.9 |
| 0.8 | 62.1 |
| 0.9 | 62.7 |
| 1 | 63.2 |
| 1.1 | 63.4 |
| 1.2 | 61.9 |
| 1.3 | 61.5 |
| 1.4 | 61.2 |
| 1.5 | 60.8 |
| 1.6 | 60.6 |
| 1.7 | 60.5 |
| 1.8 | 60.3 |
| 1.9 | 60.1 |
| 2 | 59.7 |
| 2.2 | 59.1 |
| 2.4 | 58.4 |
| 2.6 | 58.2 |
| 2.8 | 57.1 |
| 3 | 55.2 |
| 3.1 | 24.3 |
| 3.3 | 22.1 |
| 3.5 | 20.6 |
| 3.7 | 18.9 |
| 4 | 16.8 |
| 4.5 | 15.2 |
| 5 | 14.1 |
| 6 | 13.1 |
| 7 | 11.0 |
| 8 | 10.2 |
| 10 | 9.3 |
| 15 | 8.2 |
| 20 | 7.5 |
| 30 | 6.5 |

As shown in Table 4 above, when the content of MC was less than 0.03 parts by weight and more than 3.0 parts by weight, the short-axis change rate was high and the maximum breaking force (N) was low. Thus, in this case, the "maximum breaking force (N)/short-axis change rate" value was low, and hence the shape retainability was not excellent. This means that the shape is easily changed even with a slight force, and this change is very unfavorable during a medical procedure for application of the bone graft composition.

When MC was contained in an amount of 0.03 to 3 parts by weight based on 1 part by weight of the bone graft material, it was possible to obtain a bone graft composition having excellent shape retainability due to a "maximum breaking force (N)/short-axis change rate" of 50 or more.

As described above, the bone graft composition according to the present disclosure has a viscosity adjusted depending on the amount of the additive, and also has excellent effects in terms of activation of bone formation, biocompatibility, and ease of use due to excellent shape retainability thereof.

The above description is merely illustrative of the technical idea of the present disclosure, and those skilled in the art to which the present disclosure pertains will appreciate that various modifications and variations are possible without departing from the essential characteristics of the present disclosure.

Accordingly, the embodiments disclosed in the present disclosure are considered to be illustrative in all respects and not restrictive, and the scope of the technical spirit of the present disclosure is not limited by these embodiments. The protection scope of the present disclosure should be interpreted by the following claims, and all technical ideas within the scope equivalent thereto should be construed as being included within the scope of the present disclosure.

## Claims

1. A bone graft composition containing a bone graft material and an additive and having adjusted viscosity,
wherein the additive comprises only a methylcellulose-based additive, and a viscosity of the bone graft composition is 100 to 500,000 centipoises (cps).

2. The bone graft composition of claim 1, wherein the methylcellulose-based additive is one or more of methylcellulose, carboxymethyl cellulose, and hydroxypropyl methylcellulose.

3. The bone graft composition of claim 1, wherein the methylcellulose-based additive is hydroxypropyl methylcellulose.

4. The bone graft composition of claim 1, wherein the methylcellulose-based additive comprises only one of methylcellulose, carboxymethyl cellulose, and hydroxypropyl methylcellulose, and the additive is contained in an amount of 0.03 to 3 parts by weight based on 1 part by weight of the bone graft material so that the viscosity of the bone graft composition is 100 to 500,000 cps.

5. The bone graft composition of claim 4, wherein the methylcellulose-based additive is hydroxypropyl methylcellulose.

6. A bone graft composition containing a bone graft material and an additive and having adjusted viscosity, wherein the additive comprises one of methylcellulose, carboxymethyl cellulose, and hydroxypropyl methylcellulose, and a viscosity of the bone graft composition is adjusted depending on an amount of the additive and is 100 to 500,000 centipoise (cps).

7. The bone graft composition of claim 6, wherein the additive is hydroxypropyl methylcellulose.

8. The bone graft composition of claim 1, wherein the additive is contained in an amount of 0.03 to 3 parts by weight based on 1 part by weight of the bone graft material so that the viscosity of the bone graft composition is 100 to 500,000 cps.

9. A bone graft composition containing a bone graft material and an additive and having adjusted viscosity, the composition having shape retainability of 50 or more, wherein the shape retainability is defined as a value obtained by dividing a maximum breaking force (Nmax) by a short-axis change rate, in which the maximum breaking force is a force at which a change in the shape of a sphere-shaped material is initiated by applying a force (N) to one side of the sphere-shaped material, and the short-axis change rate is the ratio (D-S/D) of the reduction in short-axis length (S) of the sphere-shaped material, measured after the change in the shape occurred, to the diameter (D) of the sphere-shaped material, and wherein the viscosity of the bone graft composition is adjusted depending on the amount of the additive and is 100 to 500,000 centipoise (cps).

10. The bone graft composition of claim 9, wherein the additive is hydroxypropyl methylcellulose.
